Europäisches Patentamt

European Patent Office

Office européen des brevets

(19)

(11) Publication number: **0 143 340 B2**

# NEW EUROPEAN PATENT SPECIFICATION

(12)

(45) Date of publication of the new patent specification : 20.02.91 Bulletin 91/08

(51) Int. Cl.⁵: **A61M 1/00**, A61M 1/14

(21) Application number : 84112914.1

(22) Date of filing : 26.10.84

(54) Arrangement for the venting of a liquid.

(30) Priority : 29.11.83 SE 8306573

(43) Date of publication of application :
05.06.85 Bulletin 85/23

(45) Publication of the grant of the patent :
08.06.88 Bulletin 88/23

(45) Mention of the opposition decision :
20.02.91 Bulletin 91/08

(84) Designated Contracting States :
BE CH DE FR GB IT LI LU NL

(56) References cited :
EP-A- 0 082 721
DE-A- 2 453 839
DE-A- 2 838 414
DE-A- 3 215 003
FR-A- 2 339 406
US-A- 3 878 095

(56) References cited :
US-A- 4 021 341
US-A- 4 162 974
Bedienungsanleitung Hämodialysegerät
A2008C, 1980, Fresenius AG, 6380 Homburg
v.d.H., DE

(73) Proprietor : Gambro Lundia AB
Box 10101
S-220 10 Lund (SE)

(72) Inventor : Brorsson, Fritz Lennart
Jasminvägen 7
S-240 32 Flyinge (SE)
Inventor : Dahlberg, Bengt Ake Gustav
Masvägen 8 C
S-222 33 Lund (SE)
Inventor : Holmberg, Bengt Magnus
Studentvägen 10
S-237 00 Bjärred (SE)

(74) Representative : Boberg, Nils Gunnar Erik
Gambro AB Patent Department Box 10101
S-220 10 Lund (SE)

EP 0 143 340 B2

## Description

### TECHNICAL FIELD

The present invention relates to an arrangement for the venting of a liquid which flows through a duct comprising a throttling means, a suction pump arranged downstream of it and a venting chamber arranged downstream of the pump an expansion chamber being arranged between the throttling means and the pump, and the inlet to the expansion chamber being arranged at its base and the outlet of the same at its top.

The invention is intended in particular for application in connection with an arrangement for the preparation of dialysis solution and the feed of this to a dialyser. For those versed in the art it will be clear, however, that the invention can also be applied more generally.

### BACKGROUND ART

In the American patent specifications 4 158 034 and 4 293 409, for example, an arrangement is described which is intended for the preparation and feed of dialysis fluid to a dialyser. The present invention relates to a further development and improvement of the system in accordance with these patents. One improvement of said arrangements is that an expansion chamber has been arranged between the throttling means and the pump and that the inlet to the expansion chamber has been arranged at its base and the outlet of the same at its top. Owing to this expansion chamber time is gained for the formation of bubbles of an appropriate size, which can be passed through the pump to the venting chamber without excessively being broken down in the pump. Such an expansion chamber is, however, known through figure 18 of an operating manual for the dialysis machine A 2008 C manufactured by the German firm Fresenius AG. The expansion chamber shown in said operating manual is, however, difficult to empty. It cannot for instance be emptied by means of a pump arranged further on in the system.

### DISCLOSURE OF INVENTION

A further improvement is brought about with the help of a shunt line arranged in parallel with the expansion chamber. This shunt line makes possible a complete emptying of the expansion chamber with the help of a pump arranged further on in the system.

In the shunt line a valve is suitably provided which is adapted so that it is kept shut at normal working pressure, but is opened when the normally negative pressure rises to nominally lower values, e.g. in connection with air entering into the expansion chamber when the system is emptied with the help of the abovementioned pump arranged further on in the system. The valve provided in the shunt line may simply be constituted of a compressible tube which is automatically sucked together at a nominally higher partial vacuum, that is to say the pressure corresponding to the normal working pressure.

To ensure a safe shutting off of the tube a pinch device may be arranged on the same which is adapted so as to press together the points which are to form the end points of the compressed tube already in the open position of the tube. Without this pinch device there is a danger of a certain leakage occurring just at these end points.

The inlet to the venting chamber is appropriately arranged tangentially at a hidher level than the outlet of the same. In this manner the bubble separation is facilitated, by the difference in level as well as by the centrifugal forces which arise.

In a manner known in itself an outlet is arranged in the venting chamber at is highest point for air accumulated therein together with a float valve which is adapted so as to prevent any liquid being sucked out through the air outlet.

The throttling means provided upstream of the expansion chamber is appropriately adjustable. In this way, among other things, the degree of venting can be regulated and at the same time the dialysis flow can be modified.

To allow the effective emptying of the system as a whole it is provided appropriately with a suction pump arranged downstream of the venting chamber. This pump may be constituted, for example, of the pump which normally sucks dialysis fluid through the dialyser.

The invention is thus intended in the first place, as mentioned above, to form part of a system for the preparation of dialysis fluid through mixing of one or more concentrates with water. It will be described, therefore, in the following with reference to just such a system.

The bubble separation is facilitated further if the outlet is arranged tangentially but in countercurrent to the vortex formation produced by the inlet.

### BRIEF DESCRIPTION OF DRAWINGS

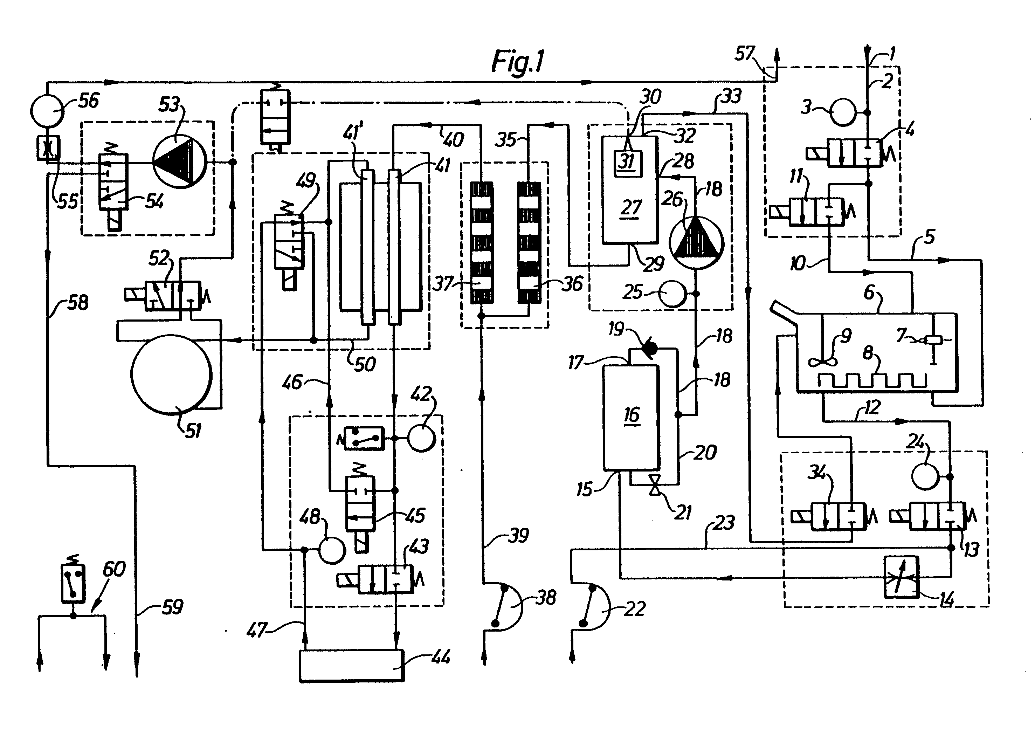

Fig. 1 shows schematically a flow diagram of a substantially complete system for the preparation of dialysis fluid.

Fig. 2 shows in greater detail and as an example a possible design of an air separation chamber forming part of the said system.

Fig. 3 shows schematically a section III-III through the same air separation chamber and is intended to illustrate the placing of the inlet and outlet respectively of the chamber.

Fig. 4 shows how a tube forming part of the system may possibly be sucked together incompletely if it is not provided with a pinch device in accordance with fig. 5 which has the effect that the sucking together will instead be as shown in fig. 6.

## BEST MODE OF CARRYING OUT THE INVENTION

In the system for the preparation of dialysis solution shown as an example the water is introduced at an inlet 1 into a duct 2 with a temperature measuring instrument 3 and an inlet valve 4. From the inlet valve 4 the water flows through a duct 5 to a heating vessel 6 with a level control float 7, a heating coil 8 and a stirrer 9. Alternatively the water can flow to the heating vessel through a duct 10 with a valve 11 which allows a greater flow so that an effective rinsing of the vessel can be achieved, e.g. in connection with sterilization.

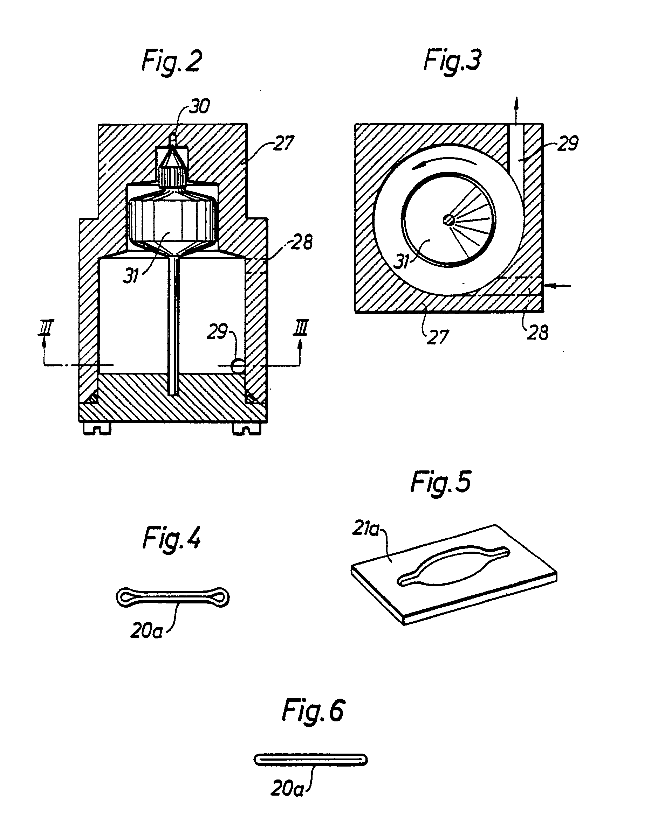

From the vessel 6 the water flows via a duct 12, a flow valve 13 with an appropriately adjustable second flow valve or throttling means 14 to an inlet 15 arranged at the base of an expansion chamber 16. At the top of this chamber is arranged an outlet 17 to a duct 18 with a nonreturn valve 19. Parallel with the chamber, moreover, a shunt line 20 with a valve 21 is arranged. In a preferred embodiment this shunt line 20 and the valve 21 are constituted quite simply of the tube 20a with a pinch device 21a as shown in fig. 4-6. Thanks to this pinch device 21a the tube at a sufficient partial pressure is given the shape obtained in fig. 6 instead of the shape as shown in fig. 4 otherwise possible.

Before the liquid has reached the expansion chamber 16 concentrate is fed to it with the help of a pump through a duct 23 and prior to this, moreover, the temperature is measured once more with the help of a temperature measuring instrument 24.

The line 18 continues via a pressure gauge 25 and a pump 26 to a venting chamber 27. An example of such a chamber is shown in more detail in fig. 2 and schematically in fig. 3. The chamber 27 is thus provided with an inlet 28 and an outlet 29 arranged at a lower level for the liquid as well as an outlet 30 for the air accumulated in the chamber. A float 31 is arranged, moreover, in the chamber which closes the outlet 30 when the liquid level has risen so high that there is a risk of the liquid being sucked out through the outlet 30.

From the venting chamber 27, via a further outlet 32, extends a duct 33 via a valve 34 back to the heating vessel 6. This duct is intended to be used in connection with sterilization in a manner which is described in more detail in the abovementioned American patent 4 158 034.

From the venting chamber 27 the liquid proceeds via a duct 35 to a conductivity meter 36 and, in case of a second concentrate having to be added, a further conductivity meter 37. The second concentrate is fed in such a case with the help of a pump 38 through a duct 39. Owing to these two conductivity meters 36 and 37 the conductivity can be measured before and after the addition of the second concentrate. The liquid is then passed through a duct 40 via a flow meter 41, the temperature measuring instrument 42 and a valve 43 to the dialyser 44 indicated schematically. If for example the temperature or the conductivity do not have the desired values, the liquid is conducted instead with the help of a valve 45 into a duct 46 past the dialyser to a second conduit 41' in the aforementioned flow meter.

If on the other hand the desired conditions are met, the liquid is passed from the dialyser 44 through a duct 47 via a pressure gauge 48 and a valve 49 to the conduit 41' in the flow meter. From there the liquid is conducted via a duct 50 with a blood leakage detector 51 and a valve 52 to a suction pump 53. Thus the dialysis fluid is sucked through the dialyser 44 with the help of this suction pump. The same pump is also used for the emptying of the system as a whole. From the pump 53 the liquid is conducted normally via an outlet valve 54, a throttling means 55 and a safety control meter 56 for pH measurement to the outlet 57. Alternatively, for example in sequence dialysis, the ultrafiltrate sucked out may be passed from the valve 54 via a duct 58 to a special collecting point 59 for such filtrate.

A safety bypass designated 60 finally is used only in connection with sterilization of the arrangement. This may be of the type, for example, which is described in more detail in American patent 4 122 010.

Naturally the invention is not limited solely to the embodiment described above, but may be modified within the scope of the following claims. Thus the parts included in the system can be modified, of course, in respect of form as well as function. Furthermore the invention can be applied, for example, also to the venting of replacement liquid for haemofiltration or plasmafers or for the venting of liquids in general. Reference is made, moreover, to the design according to EP-A-0 143 341 submitted at the same time which describes an alternative venting arrangement including parts which can be combined with the design according to the present invention.

## Claims

1. An arrangement for the venting of a liquid which flows through a duct (2, 5, 12, 18, 35) comprising a throttling means (14), a suction pump (26) arranged downstream of it and a venting chamber (27) arranged downstream of the pump (26), an expansion chamber (16) being arranged between the throttling means (14) and the pump (26), and the inlet (15) to the expansion chamber (16) being arranged at its base and the outlet (17) of the same at its top, **characterized** by a shunt line (20) arranged in parallel with the expansion chamber (16).

2. An arrangement in accordance with claim 1, **characterized** by a nonreturn valve (19) arranged directly after the outlet (17) of the expansion chamber (16).

3. An arrangement in accordance with claim 1, **characterized** by a valve (21) arranged in the shunt line (20) which is adapted so that it is kept shut at normal working pressure but is opened when the normally negative pressure rises to nominally lower values.

4. An arrangement in accordance with claim 3, **characterized** in that the valve (21) arranged in the shunt line is constituted of a compressible tube (20a) which is automatically sucked together at nominally higher partial vacuum.

5. An arrangement in accordance with claim 4, **characterized** by a pinch device (21a) arranged on the tube (20a) which is adapted so that it compresses the points which are to form the end points of the compressed tube already in the open position of the tube.

6. An arrangement in accordance with anyone of claims 1-5, **characterized** in that the inlet (28) to the venting chamber (27) is arranged tangentially to the venting chamber on a higher level that the outlet (29) of the same.

7. An arrangement in accordance with claim 6, **characterized** in that the outlet (29) of the venting chamber is also arranged tangentially to the venting chamber but in countercurrent ot the vortex formation produced by the inlet (28).

8. An arrangement in accordance with anyone of claims 1-7, **characterized** by an air outlet (30) arranged at the highest point of the venting chamber (27) for air accumulated therein, and by a float valve (31) arranged in the same chamber (27) which is adapted so as to prevent liquid from being sucked out through the air outlet (30).

9. An arrangement in accordance with anyone of the preceding claims, **characterized** in that the throttling means (14) arranged upstream of the expansion chamber (16) is adjustable.

10. An arrangement in accordance with anyone of the preceding claims, **characterized** by a suction pump (53) arranged downstream of the venting chamber (27).

11. An arrangement in accordance with anyone of the preceding claims, **characterized** in that it forms part of a system for the preparation of dialysis fluid through mixing of one or more concentrates with water.

**Ansprüche**

1. Anordnung zum Entlüften einer Flüssigkeit, die durch eine Leitung (2, 5, 12, 18, 35) fließt, mit einer Drossel (14), einer Saugpumpe (26), die stromabwärts von dieser angeordnet ist, und einer Entlüftungskammer (27), die stromabwärts der Pumpe angeordnet ist, wobei eine Expansionskammer (16) zwischen der Drossel (14) und der Pumpe (26) angeordnet ist und der Einlaß (15) zur Expansionskammer (16) an deren unteren Ende und der Auslaß (17) derselben an deren oberen Ende angeordnet sind, gekennzeichnet durch, eine Nebenleitung (20), die parallel zur Expansionskammer (16) angeordnet ist.

2. Anordnung nach Anspruch 1, gekennzeichnet durch ein Rückschlagventil (19), das unmittelbar hinter dem Auslaß (17) der Expansionskammer (16) angeordnet ist.

3. Anordnung gemäß Anspruch 2, gekennzeichnet durch ein Ventil (21), das in der Nebenleitung (20) angeordnet und so angepaßt ist, daß es bei normalem Arbeitsdruck geschlossen bleibt, aber geöffnet ist, wenn der normalerweise negative Druck zu nominell niedrigeren Werten ansteigt.

4. Anordnung gemäß Anspruch 3, dadurch gekennzeichnet, daß das in der Nebenleitung angeordnete Ventil (21) aus einem kompressiblen Schlauch (20a) gebildet ist, der bei nominell höherem Teilvakuum automatisch zusammengesaugt wird.

5. Anordnung gemäß Anspruch 4, gekennzeichnet durch eine auf dem Schlauch (20a) angeordnete Klemmapparatur (21a), die so angepaßt ist, daß sie die Stellen zusammenpreßt, die die Endstellen des zusammengepreßten Schlauches bereits in der offenen Stellung des Schlauches bilden sollen.

6. Anordnung gemäß einem der Ansprüche 1 bis 5, dadurch gekennzeichnet, daß der Einlaß (28) zur Entlüftungskammer (27) tangential zur Entlüftungskammer auf einem höheren Niveau als der Auslaß (29) derselben angeordnet ist.

7. Anordnung gemäß Anspruch 6, dadurch gekennzeichnet, daß der Auslaß (29) der Entlüftungskammer auch tangential zur Entlüftungskammer angeordnet ist, aber im Gegenstrom zu der durch den Einlaß (28) erzeugten Wirbelformation.

8. Anordnung gemäß einem der Ansprüche 1 bis 6, gekennzeichnet durch einem Luftauslaß (30), der am höchsten Punkt der Entlüftungskammer (27) für darin angesammelte Luft angeordnet ist, und durch ein Schwimmerventil (31), das in derselben Kammer (27) angeordnet ist und so angepaßt ist, daß es das Aussaugen von Flüssigkeit durch den Luftauslaß (30) verhindert.

9. Anordnung gemäß einem der vorhergehenden Ansprüche, dadurch gekennzeichnet, daß die stromaufwärts der Expansionskammer (16) angeordnete Drossel (14) einstellbar ist.

10. Anordnung gemäß einem der vorhergehenden Ansprüche, gekennzeichnet durch eine Saugpumpe (53), die stromabwärts der Entlüftungskammer (27) angeordnete ist.

11. Anordnung gemäß einem der vorhergehenden Ansprüche, dadurch gekennzeichnet, daß sie Teil eines Systems für die Bereitung eines Dialysefluids durch Mischen von einem oder mehreren Konzentraten mit Wasser bildet.

## Revendications

1. Dispositif pour le désaérage d'un liquide qui circule dans une tuyauterie (2, 5, 12, 18, 35) comprenant un organe d'étranglement (14), une pompe d'aspiration (26) placée en aval de cet organe et une chambre de purge (27) placée en aval de la pompe (26), une chambre de détente (16) étant prévue entre l'organe d'étranglement (14) et la pompe (26), et l'entrée (15) à la chambre de détente (16) étant située à sa base et la sortie (17) de cette chambre étant située à son sommet, caractérisé en ce qu'une tuyauterie de dérivation (20) est disposée en parallèle avec la chambre de détente (16).

2. Dispositif suivant la revendication 1, caractérisé en ce qu' un clapet de non retour (19) est placé directement après la sortie (17) de la chambre de détente (16).

3. Dispositif suivant la revendication 1, caractérisé en ce qu'une valve (21) est placée dans la tuyauterie de dérivation (20) et elle est conçue de façon à rester fermée à la pression normale de travail mais à s'ouvrir lorsque la pression normalement négative augmente à des valeurs nominalement inférieures.

4. Dispositif suivant la revendication 3, caractérisé en ce que la valve (21) placée dans la tuyauterie de dérivation est constituée d'un tube compressible (20a) qui est automatiquement aspiré sur lui-même, à un vide partiel nominalement plus grand.

5. Dispositif suivant la revendication 4, caractérisé en ce qu'un dispositif de pincement (21a) placé sur le tube (20a) est prévu de sorte que, déjà dans la position ouverte du tube, il comprime les points qui doivent former les points d' extrémité du tube comprimé.

6. Dispositif suivant l'une quelconque des revendications 1 à 5, caractérisé en ce que l'entrée (28) à la chambre de purge (27) est disposée tangentiellement à la chambre de purge, à un niveau plus élevé que celui de la sortie (29) de cette chambre.

7. Dispositif suivant la revendication 6, caractérisé en ce que la sortie (29) de la chambre de purge est également disposée tangentiellement à la chambre de purge mais à contre-courant du vortex engendré par l'entrée (28).

8. Dispositif suivant l'une quelconque des revendications 1 à 7, caractérisé en ce qu'une sortie d'air (30) est prévue au point le plus haut de la chambre de purge (27), pour l'évacuation de l'air accumulé dans cette chambre, et en ce qu'une soupape à flotteur (31) est placée dans la même chambre (27) et elle est conçue pour empêcher l'aspiration du liquide par la sortie d'air (30).

9. Dispositif suivant l'une quelconque des revendications précédentes, caractérisé en ce que l'organe d'étranglement (14) placé en amont de la chambre de détente (16) est réglable.

10. Dispositif suivant l'une quelconque des revendications précédentes, caractérisé en ce qu'une pompe d'aspiration (53) est placée en aval de la chambre de purge (27).

11. Dispositif suivant l'une quelconque des revendications précédentes, caractérisé en ce qu'il fait partie d'un système pour la préparation de fluide de dialyse par mélange d'un ou plusieurs produits concentrés avec de l'eau

5

Fig.1

Fig.2

Fig.3

Fig.5

Fig.4

Fig.6